(19) Europäisches Patentamt
European Patent Office
Office européen des brevets

(11) **EP 1 824 807 B2**

(12) **NEUE EUROPÄISCHE PATENTSCHRIFT**
Nach dem Einspruchsverfahren

(45) Veröffentlichungstag und Bekanntmachung des Hinweises auf die Entscheidung über den Einspruch:
**08.02.2017 Patentblatt 2017/06**

(45) Hinweis auf die Patenterteilung:
**03.11.2010 Patentblatt 2010/44**

(21) Anmeldenummer: 05825969.8

(22) Anmeldetag: **09.12.2005**

(51) Int Cl.:
*B01D 3/00* (2006.01)   *C07C 41/56* (2006.01)
*C07C 41/58* (2006.01)   *C07C 43/303* (2006.01)

(86) Internationale Anmeldenummer:
**PCT/EP2005/013193**

(87) Internationale Veröffentlichungsnummer:
**WO 2006/061231 (15.06.2006 Gazette 2006/24)**

(54) **VERFAHREN ZUR HERSTELLUNG VON ACETALEN**

METHOD FOR THE PRODUCTION OF ACETALS

PROCEDE DE PRODUCTION D'ACETALS

(84) Benannte Vertragsstaaten:
**AT BE BG CH CY CZ DE DK EE ES FI FR GB GR HU IE IS IT LI LT LU LV MC NL PL PT RO SE SI SK TR**

(30) Priorität: **09.12.2004 DE 102004059334**

(43) Veröffentlichungstag der Anmeldung:
**29.08.2007 Patentblatt 2007/35**

(73) Patentinhaber: **Ticona GmbH**
**65451 Kelsterbach (DE)**

(72) Erfinder:
• **GÖRING, Matthias**
**65719 Hofheim (DE)**
• **HOFFMOCKEL, Michael**
**65527 Niedernhausen (DE)**
• **LINGNAU, Jürgen**
**55130 Mainz-Laubenheim (DE)**
• **MUECK, Karl-Friedrich**
**65207 Wiesbaden (DE)**

(74) Vertreter: **Schön, Christoph**
**Dr. Schön, Neymeyr & Partner mbB**
**Bavariaring 26**
**80336 München (DE)**

(56) Entgegenhaltungen:
CH-A5- 688 041        DE-B- 1 002 305
US-A- 4 967 014       US-A1- 2002 035 298
US-A1- 2003 069 451   US-B1- 6 379 507

• OTHMER D. ET AL: 'Industrial and Engineering Chemistry' IND. ENG. CHEM. Bd. 38, Nr. 12, 1946, Seite 1230

EP 1 824 807 B2

**Beschreibung**

Die Erfindung betrifft ein verbessertes Verfahren zur Herstellung von Acetalen, insbesondere wasser- und alkoholfreien Acetalen. Acetale sind Lösungsmittel und Zwischenprodukte in der chemischen Industrie.

Acetale lassen sich durch säurekatalysierte Umsetzung von Alkoholen mit Aldehyden herstellen. Ein industriell wichtiges Acetal ist Methylal (Formaldehyddimethylacetal). Es wird technisch durch säurekatalysierte Reaktion von wässrigem Formaldehyd mit Methanol hergestellt. Aus dem Reaktionsgemisch läßt sich Methylal durch Destillation abtrennen, wobei es aber von Wasser und Methanol begleitet wird, weil beide Komponenten jeweils ein binäres Azeotrop mit Methylal bilden. Für die Lösung dieses Trennproblems wurden zahlreiche Verfahren vorgeschlagen, die meisten von Ihnen nutzen die Extraktion oder Extraktivrektifikation, um die Azeotrope zu überwinden.

Die DE 1 002 305 offenbart ein Verfahren zur Extraktivdestillation eines wasser- und methanolhaltigen Methylals mit Wasser, um ein methanolfreies Methylal zu gewinnen.

Die CH 688 041 beschreibt ein Verfahren zur Herstellung von Methylal aus Formaldehyd und Methanol durch Reaktivdestillation. Die Reaktion geschieht in einer Destillationskolonne unter Katalyse eines sauren Ionentauscherharzes, welches in geeigneter Form in einen oder mehrere Kolonnenschüsse eingebracht wurde. Am Kopf der Kolonne erhält man das Azeotrop aus Methylal und Methanol, welches in den weiteren drei Trennschritten

- Extraktion mit wässriger Lauge,
- Rektifikation zum Azeotrop mit 0,9 - 1 % Wasser,
- abschließende Trocknung vom Wasser mit einem anorganischen Trocknungsmittel wie Calciumchlorid, Zeolithen oder Silikagel,

zu einem ca. 99,99% reinen Methylal aufgearbeitet wird. Das Verfahren hat den Nachteil, dass für die alkalischen Extraktion und für die abschließende Trocknung Hilfsstoffe eingeführt werden müssen, die entsorgt oder aufgearbeitet werden müssen. Der an sich vorteilhaften Reaktivdestillation folgen drei weitere Trennschritte bis zum Reinprodukt.

Die US 6 379 507 offenbart ein Verfahren zur Herstellung von Methylal aus Formaldehyd und Methanol, demgemäß an mindestens vier Positionen einer Destillationskolonne das jeweilige Gemisch aus Wasser, Formaldehyd, Methanol und Methylal entnommen wird und die Gemische jeweils in einer erzwungenen Strömung den korrespondierenden mindestens vier Festbettreaktoren zugeführt werden. Die mit einem sauren Ionentauscherharz beschickten Festbettreaktoren sind räumlich von der Kolonne getrennte, eigene Behälter. Der apparative Aufwand für mehrere separate Reaktoren mit eigener Pumpe und Regelung ist beträchtlich. Das Verfahren erreicht eine Konzentration von 0,3 - 0,4 % Methanol und 1,2 % Wasser am Kolonnenkopf, allerdings nur unter Verwendung eines Entschäumers, der auf den Kolonnenkopf geführt wird.

Die US 6 015 875 beschreibt ein Verfahren zur Herstellung von Acetalen aus Formaldehyd und Methanol durch Reaktivdestillation bei erhöhter Temperatur und ca. 7 bar Druck, wobei Zeolithe als saure Katalysatoren eingesetzt werden. Das Verfahren erzielt jedoch nur 73 - 77 % Methylal am Kopf der Reaktivkolonne.

Es besteht daher die Aufgabe, ein verbessertes Verfahren für die Herstellung von Acetalen zu finden, welches

- möglichst wenige Trennschritte benötigt,
- möglichst wenig apparativen Aufwand erfordert,
- keinen unverhältnismäßigen Zusatzaufwand durch die Einführung wässriger Alkalien als Extraktionsmittel oder öliger Entschäumer nach sich zieht
- bereits im ersten Schritt eine möglichst hohe Produktkonzentration des Acetals liefert.

Überraschend wurde gefunden, dass Acetale in hoher Produktkonzentration und Ausbeute entstehen, wenn man die Reaktivdestillation und die Extraktivdestillation in einer Kolonne kombiniert. Dieses Verfahren liefert selbst dann gute Ergebnisse, wenn man als Extraktionsmittel ein Reaktionsprodukt, wie z.B. Wasser, verwendet, von dem der Fachmann eine Beschleunigung der Rückreaktion und damit eine Beeinträchtigung der Ausbeute erwartet.

Gegenstand der Erfindung ist daher ein Verfahren zur Herstellung von Acetalen aus Aldehyden und Alkoholen durch Reaktivdestillation in einer Kolonne, dadurch gekennzeichnet, dass man

    a) Aldehyd und Alkohol der Reaktions-Destillations-Zone zuführt und zumindest teilweise zum Acetal umsetzt,

    b) den unterhalb der Reaktions-Destillations-Zone angeordneten Kolonnensumpf beheizt, um das Sumpfgemisch zumindest teilweise zu verdampfen und eine definierte Temperatur in der Reaktions-Destillations-Zone einzustellen,

    c) in einen oberhalb der Reaktions-Destillations-Zone angeordneten Verstärkungsteil der Kolonne entsalztes Wasser als Extraktionsmittel einspeist und

    d) am Kopf der Kolonne ein Produkt mit mehr als 90 Gew.-% Acetal kondensiert.

In einer bevorzugten Ausführungsform des Verfahrens wird die Reaktions-Destillations-Zone erzeugt, indem man einen oder mehrere Schüsse der Kolonne mit einem sauren Festbettkatalysator beschickt.

In einer weiteren bevorzugten Ausführungsform des Verfahrens wird in der Kolonne ein Rücklaufverhältnis zwischen R = 0,5 und R = 10 eingestellt.

In einer weiteren bevorzugten Ausführungsform des Verfahrens wird das im Schritt d) des Verfahrens anfallende Kopfprodukt in nicht mehr als zwei nachfolgenden Trennoperationen auf einen Gehalt von mindestens 99 Gew.-% Acetal aufkonzentriert.

Zur Durchführung des erfindungsgemäßen Verfahrens eignet sich grundsätzlich jede Destillationskolonne ohne besondere apparative Anforderungen, die in einem oder mehreren ihrer Schüsse mit einem sauren Festbettkatalysator beschickt wurde. Als saure Katalysatoren eignen sich saure ionentauscherharze, Zeolithe oder auch trägergebundene Heteropolysäuren. Bevorzugt sind saure ionentauscherharze.

Das erfindungsgemäße Verfahren ist zur Herstellung beliebiger Acetale durch Umsetzung geeigneter Alkohole und Aldeyhde, vorzugsweise von primären aliphatischen Alkoholen mit ein bis sechs Kohlenstoffatomen mit aliphatischen oder aromatischen Aldehyden, geeignet. Bevorzugt sind die Ausgangsstoffe mit einem oder zwei Kohlenstoffatomen, nämlich Methanol und Ethanol sowie Formaldehyd und Acetaldehyd. Besonders bevorzugt ist die Herstellung von Methylal aus Formaldehyd und Methanol. Im folgenden wird das Verfahren anhand dieses bevorzugten Beispiels erläutert.

Formaldehyd wird in Form einer wässrigen Lösung der Umsetzung zugeführt. Die Konzentration der Lösung liegt im Bereich von 5 bis 75 Gew.-% bevorzugt 15 bis 55 Gew.-% und besonders bevorzugt von 18 bis 40 Gew.-%. Die Formaldehydlösung kann zur Stabilisierung bereits Methanol in Konzentrationen von 1 bis 20 Gew.-% enthalten. Diese Methanolmenge wird bei der Berechnung der stöchiometrischen Verhältnisse berücksichtigt. Bevorzugt ist die Formaldehydlösung frei von Metallkationen, weil die Anwesenheit von Metallkationen zu einer beschleunigten Desaktivierung des Katalysators führen kann.

In einer bevorzugten Ausführungsform der Erfindung werden die Edukte nicht direkt der Reaktions-Destillations-Zone in der Kolonne zugeführt, sondern werden zunächst gemischt und passieren einen Vorreaktor. Der Vorreaktor ist mit dem sauren Katalysator bestückt und kann beispielsweise ein Rohrreaktor sein. Im Vorreaktor werden Aldehyd und Alkohol bereits teilweise umgesetzt, so dass ein Gemisch aus Edukten und dem Acetal sowie gegebenenfalls Wasser den Vorreaktor verläßt und der Reaktions-Destillations-Zone der Kolonne zugeführt wird. Der Umsatz im Vorreaktor bezogen auf das im stöchiometrischen Unterschuss eingesetzte Edukt beträgt 10 - 50 %, bevorzugt 15 - 40 %.

Das molare Verhältnis von Aldehyd und Alkohol kann in einem relativ breiten Bereich um die stöchiometrische Äquivalenz von Aldehyd : Alkohol = 0,5 variieren. Wenn man die Möglichkeit hat, eines der beiden Edukte in einem Verbundprozess zu verwenden oder aufzuarbeiten, kann man mittels eines stöchiometrischen Überschusses dieses Edukts das jeweilig andere Edukt praktisch vollständig umsetzen. In bevorzugten Ausführungsformen der Erfindung liegt das molare Verhältnis Aldehyd : Alkohol zwischen 0,25 und 1,0. Der Gesamtumsatz in Vorreaktor und Reaktivkolonne beträgt im erfindungsgemäßen Verfahren mehr als 80 %, bevorzugt mehr als 90 % und besonders bevorzugt mehr als 95 %, bezogen auf das im stöchiometrischen Unterschuss eingesetzte Edukt.

Für die Herstellung von Methylal aus Formaldehyd und Methanol wird die Reaktivkolonne nahe Normaldruck, bevorzugt bei 20 - 250 mbarü betrieben. Die Sumpfbeheizung wird so geregelt, dass sich eine Sumpftemperatur von 95 - 105 °C einstellt. Die Temperatur in der Reaktions-Destillations-Zone beträgt 50 - 90 °C. Die Temperatur am Kolonnenkopf beträgt 40 - 50 °C.

Der oberhalb der Reaktions-Destillations-Zone angeordnete Verstärkungsteil der Kolonne kann nach dem Stand der Technik als Bodenkolonne, beispielsweise als Siebboden- oder Glockenbodenkolonne oder als gepackte Kolonne mit einer geordneten oder einer Füllkörperpackung ausgeführt sein. In den Verstärkungsteil der Kolonne wird ein Extraktionsmittel eingespeist, um den Alkohol in dem aufsteigenden Dampfgemisch abzureichern. Über einen geeigneten Zulaufverteiler wird sichergestellt, dass das Extraktionsmittel über den gesamten Kolonnenquerschnitt verteilt wird und ein guter Stoffaustausch mit dem aufsteigenden Dampf eintritt. In der bevorzugten Herstellung von Methylal aus Methanol und Formaldehyd ist das Extraktionsmittel entsalztes Wasser ist das Extraktionsmittel entsalztes Wasser.

Der über den Kolonnenkopf austretende Brüdenstrom wird vollständig kondensiert und teilweise wieder in die Kolonne oberhalb des Verstärkungsteil eingespeist. Das Rücklaufverhältnis R der Kolonne, wobei R durch das folgende Verhältnis definiert ist,

$$R = \text{Rücklauf} / \text{Entnahme}$$

wird nach den Anforderungen an das Verfahren eingestellt. Ein hohes Rücklaufverhältnis ergibt eine höhere Reinheit des Kopfproduktes, resultiert aber in einem höheren spezifischen Energieverbrauch. R liegt im erfindungsgemäßen Verfahren zwischen 0,5 und 10, bevorzugt zwischen 1 und 8 und besonders bevorzugt zwischen 2 und 6.

Die Reinheit des Acetals am Kopf der Reaktiv-Kolonne gemäß der Erfindung beträgt mehr als 90 %. Bei der Herstellung von Methylal aus Formaldehyd und Methanol beträgt die Kopfkonzentration bevorzugt mehr als 95 Gew.-% Methylal sowie weniger als 2 Gew.-% Methanol und weniger als 3 Gew.-% Wasser. Besonders bevorzugt sind die Kopfkonzentrationen über 97 Gew.-% Methylal mit unter 1 Gew.-% Methanol und unter 2 Gew.-% Wasser. Ganz besonders bevorzugt sind über 98 Gew.-% Methylal mit unter 0,5 Gew.-% Methanol und unter 1 Gew.-% Wasser. Die Konzentration von Formaldehyd im Kopfprodukt liegt erfindungsgemäß unter 0,25 Gew.-%, bevorzugt unter 0,15 Gew.-% und besonders bevorzugt unter 0,10 Gew.-%.

Das so erhaltene Kopfprodukt wird in nicht mehr als zwei folgenden Trennschritten auf eine Reinheit von mindestens 99 % gereinigt. Dies können beispielsweise eine Extraktivrektifikation zur Entfernung des Wassers und eines Teils des Methanols sein. Für hohe Reinheitsanforderungen kann eine Destillation zur Entfernung von Leichtsiedern angeschlossen werden. Es ist auch möglich, zuerst die Leichtsieder durch Destillation zu entfernen und danach eine Extraktivrektifikation anzuschließen. Es sind auch Kombinationen mit anderen Grundoperationen wie Extraktion oder Trocknung möglich.

In einer bevorzugten Ausführungsform der Erfindung wird Methylal von Leichtsiedern durch Destillation befreit und restliches Wasser und Methanol durch eine Extraktivrektifikation entfernt. Das Extraktionsmittel ist schwerflüchtig und wassermischbar, beispielsweise ein mehrwertiger Alkohol mit 2-4 Kohlenstoffatomen, bevorzugt Ethylenglykol. Bevorzugt hat das Acetal-Reinprodukt nach den nicht mehr als zwei Trennschritten eine Reinheit von mindestens 99,9 %.

Weitere bevorzugte Ausgestaltungsformen der Erfindung ergeben sich aus den Unteransprüchen.

**Beispiel:**

Die Erfindung wird anhand eines Ausführungsbeispiels und einer Figur verdeutlicht. In einer Kolonne mit 350 mm Durchmesser werden die Schüsse 3, 4, und 5 mit Katapak-Elementen (Produkt der Firma Sulzer Chemtech) bestückt, deren Taschen mit einem sauren Ionentauscherharz befüllt sind. Zwischen die Schüsse 4 und 5 wird der Ablauf eines ebenfalls mit saurem Ionentauscherharz befüllten Vorreaktors eingespeist. Dem Vorreaktor werden 280 kg/h einer 20 %igen wässrigen Formaldehydlösung gemeinsam mit 100 kg/h Methanol zugeführt. Der Vorreaktor wird auf 80 °C temperiert. Sein Ablauf setzt sich zusammen aus 19.5% Methanol, 11.5% Formaldehyd, 8 % Methylal und 61% Wasser, entsprechend einem Umsatz von 26 % des Methanols im Vorreaktor. Der Kolonnensumpf wird mit ca. 120 kg/h Niederdruckdampf beheizt, so dass sich im Sumpf eine Temperatur von 100°C, im Katapak-Bett eine Temperatur von 50°C und am Kolonnenkopf eine Temperatur von 46°C einstellt. Zwischen Schuss 5 und 6 der Reaktionskolonne werden 100 kg/h entsalztes Wasser über einen Verteilerboden eingespeist. Der Brüden der Kolonne wird vollständig kondensiert, 500 kg/h des Destillats werden als Rücklauf auf den Kopf der Kolonne zurückgefahren und 110 kg/ Stunde Rohmethylal entnommen. Das Rohmethylal hat eine Zusammensetzung von 98,2 % Methylal, 0,4 % Methanol, 0,8 % Wasser und 0,6 % Methylformiat. Der Sumpfstrom der Reaktivkolonne von 380 kg/h enthält 1 % Methanol und 4% Formaldehyd. Der Umsatz bezogen auf Methanol beträgt 95,5%.

Nach einer anschließenden Destillation der Leichtsieder und einer Extraktivrektifikation mit Ethylenglykol erhält man ein Reinmethylal von über 99,9% Reinheit, welches unter 100 ppm Methanol, unter 50 ppm Wasser und unter 100 ppm Methylformiat enthält.

**Patentansprüche**

1. Verfahren zur Herstellung von Acetalen aus Aldehyden und Alkoholen durch Reaktivdestillation, **dadurch gekennzeichnet, dass**

   a) Aldehyd und Alkohol der Reaktions-Destillations-Zone einer Destillationskolonne zugeführt und zumindest teilweise zum Acetal umgesetzt werden,
   b) der unterhalb der Reaktions-Destillations-Zone angeordnete Kolonnensumpf beheizt wird,
   c) in einen oberhalb der Reaktions-Destillations-Zone angeordneten Verstärkungsteil der Kolonne entsalztes Wasser als Extraktionsmittel eingespeist wird und
   d) am Kopf der Kolonne ein Produkt mit mehr als 90 Gew.-% Acetal kondensiert wird.

2. Verfahren nach Anspruch 1, **dadurch gekennzeichnet, dass** die Reaktions-Destillations-Zone aus einem oder mehreren Schüssen der Kolonne besteht, die mit einem sauren Festbettkatalysator beschickt sind.

3. Verfahren nach Anspruch 1 oder 2, **dadurch gekennzeichnet, dass** in der Kolonne ein Rücklaufverhältnis zwischen R = 0,5 und R = 10 eingestellt wird.

4. Verfahren nach einem oder mehreren der Ansprüche 1 bis 3, **dadurch gekennzeichnet, dass** das Kopfprodukt der Kolonne durch nicht mehr als zwei nachfolgende Trennoperationen auf einen Gehalt von mindestens 99 Gew.-% Acetal aufkonzentriert wird.

5. Verfahren nach einem oder mehreren der Ansprüche 1 bis 4, **dadurch gekennzeichnet, dass** Aldehyde und Alkohole mit einem oder zwei Kohlenstoffatomen als Edukte eingesetzt werden.

6. Verfahren nach Anspruch 5 für die Herstellung von Methylal aus Formaldehyd und Methanol.

7. Verfahren nach einem oder mehreren der Ansprüche 1 bis 6, **dadurch gekennzeichnet, dass** die Edukte gemischt und einem Vorreaktor zugeführt werden, bevor sie in die Reaktions-Destillations-Zone der Kolonne eingebracht werden.

8. Verfahren nach Anspruch 7, **dadurch gekennzeichnet, dass** der Umsatz im Vorreaktor bezogen auf das im stöchiometrischen Unterschuss eingesetzte Edukt 10 - 50 %, bevorzugt 15 - 40 % beträgt.

**9.** Verfahren nach einem oder mehreren der Ansprüche 1 bis 8, **dadurch gekennzeichnet, dass** der Umsatz, bezogen auf das im stöchiometrischen Unterschuss eingesetzte Edukt, mehr als 80 %, bevorzugt mehr als 90 % und besonders bevorzugt mehr als 95 % beträgt

**10.** Verfahren nach Anspruch 3. **dadurch gekennzeichnet, dass** das Rücklaufverhältnis R zwischen 1 und 8 und bevorzugt zwischen 2 und 6 liegt.

**11.** Verfahren nach einem oder mehreren der Ansprüche 1 bis 10, **dadurch gekennzeichnet, dass** die Kopfkonzentration mehr als 95% Acetal beträgt.

**12.** Verfahren nach einem oder mehreren der Ansprüche 1 bis 11, **dadurch gekennzeichnet, dass** die Kopfkonzentration mehr als 97% Acetal beträgt.

**13.** Verfahren nach einem oder mehreren der Ansprüche 1 bis 12, **dadurch gekennzeichnet, dass** die Kopfkonzentration mehr als 98% Acetal beträgt.

**14.** Verfahren nach Anspruch 4, **dadurch gekennzeichnet, dass** die Reinheit des Acetals nach den anschließenden nicht mehr als zwei Trennschritten mindestens 99,9% beträgt.

**Claims**

**1.** A process for preparing acetals from aldehydes and alcohols by reactive distillation, **characterized in that**

a) aldehyde and alcohol are fed to the reaction/distillation zone of a distillation column and at least partially converted to the acetal,
b) the column bottom disposed below the reaction/distillation zone is heated,
c) demineralized water as extractant is fed into a rectifying section of the column, disposed above the reaction/distillation zone, and
d) a product having more than 90% by weight of acetal is condensed at the top of the column.

**2.** The process according to claim 1, **characterized in that** the reaction/distillation zone consists of one or more sections of the column which are charged with an acidic fixed bed catalyst.

**3.** The process according to claim 1 or 2, **characterized in that** a reflux ratio between R = 0.5 and R = 10 is set in the column.

**4.** The process according to one or more of claims 1 to 3, **characterized in that** the top product of the column is concentrated to a content of at least 99% by weight of acetal by not more than two subsequent separating operations.

**5.** The process according to one or more of claims 1 to 4, **characterized in that** aldehyde and alcohols having one or two carbon atoms are used as reactants.

**6.** The process according to claim 5 for the preparation of methylal from formaldehyde and methanol.

**7.** The process according to one or more of claims 1 to 6, **characterized in that** the reactants are mixed and fed to a prereactor before being introduced into the reaction/distillation zone of the column.

**8.** The process according to claim 7, **characterized in that** the conversion in the prereactor based on the reactant used in stoichiometric deficiency is 10-50%, preferably 15-40%.

**9.** The process according to one or more of claims 1 to 8, **characterized in that** the conversion, based on the reactant used in stoichiometric deficiency, is more than 80%, preferably more than 90% and more preferably more than 95%.

**10.** The process according to claim 3, **characterized in that** the reflux ratio R is between 1 and 8 and preferably between 2 and 6.

**11.** The process according to one or more of claims 1 to 10, **characterized in that** the top concentration is more than 95% acetal.

**12.** The process according to one or more of claims 1 to 11, **characterized in that** the top concentration is more than 97% acetal.

**13.** The process according to one or more of claims 1 to 12, **characterized in that** the top concentration is more than 98% acetal.

**14.** The process according to claim 4, **characterized in that** the purity of the acetal after the subsequent not more than two separation steps is at least 99.9%.

**Revendications**

**1.** Procédé pour la préparation d'acétals à partir d'aldéhydes et d'alcools par distillation réactive, **caractérisé en ce que**

a) l'on alimente en aldéhyde et alcool une zone de réaction-distillation d'une colonne de distillation en les transformant au moins partiellement en acétal,
b) l'on chauffe le bas de colonne disposé au-dessous de la zone de réaction-distillation,
c) l'on alimente en eau déminéralisée en guise d'agent d'extraction une partie à concentration de la colonne disposée au-dessus de la zone de réaction-distillation, et
d) l'on condense à la tête de la colonne un produit avec plus de 90% en poids d'acétal.

2. Procédé selon la revendication 1, **caractérisé en ce que** la zone de réaction-distillation se compose d'une ou plusieurs sections de la colonne chargées avec un catalyseur acide en lit fixe.

3. Procédé selon la revendication 1 ou 2, **caractérisé en ce qu'**un rapport de reflux compris entre R = 0,5 et R = 10 est réglé dans la colonne.

4. Procédé selon l'une ou plusieurs des revendications 1 à 3, **caractérisé en ce que** le produit de tête de la colonne est concentré à une teneur d'au moins 99% en poids d'acétal par au plus deux opérations de séparation consécutives.

5. Procédé selon l'une ou plusieurs des revendications 1 à 4, **caractérisé en ce que** des aldéhydes et alcools avec un ou deux atomes de carbone sont utilisés en tant qu'éduits.

6. Procédé selon la revendication 5 pour la préparation de méthylal à partir de formaldéhyde et de méthanol.

7. Procédé selon l'une ou plusieurs des revendications 1 à 6, **caractérisé en ce que** les éduits sont mélangés et amenés à un préréacteur avant d'être introduits dans la zone de réaction-distillation de la colonne.

8. Procédé selon la revendication 7, **caractérisé en ce que** le taux de conversion dans le préréacteur par rapport à l'éduit utilisé en insuffisance stoechiométrique est de 10-50%, de préférence de 15-40%.

9. Procédé selon l'une ou plusieurs des revendications 1 à 8, **caractérisé en ce que** le taux de conversion par rapport à l'éduit utilisé en insuffisance stoechiométrique est de plus de 80%, de préférence de plus de 90%, et de manière particulièrement préférée, de plus de 95%.

10. Procédé selon la revendication 3, **caractérisé en ce que** le rapport de reflux R est compris entre 1 et 8, et de préférence entre 2 et 6.

11. Procédé selon l'une ou plusieurs des revendications 1 à 10, **caractérisé en ce que** la concentration de tête comporte plus de 95% d'acétal.

12. Procédé selon l'une ou plusieurs des revendications 1 à 11, **caractérisé en ce que** la concentration de tête comporte plus de 97% d'acétal.

13. Procédé selon l'une ou plusieurs des revendications 1 à 12, **caractérisé en ce que** la concentration de tête comporte plus de 98% d'acétal.

14. Procédé selon la revendication 4, **caractérisé en ce que** la pureté de l'acétal après les au plus deux opérations de séparation consécutives est au moins de 99,9%.

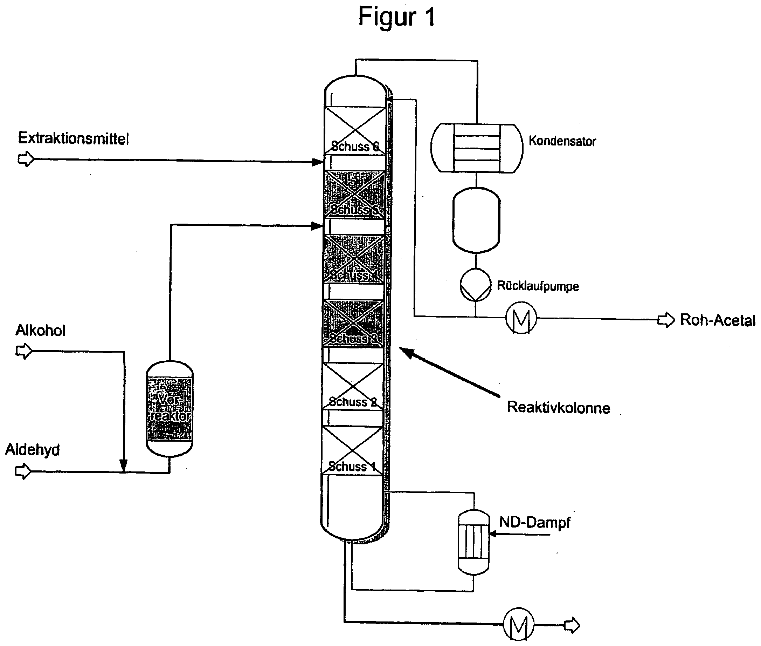

# Figur 1

EP 1 824 807 B2

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- DE 1002305
- CH 688041
- US 6379507 B
- US 6015875 A